Europäisches Patentamt

European Patent Office

Office européen des brevets

(19)

(11) EP 1 374 797 A1

(12) **EUROPEAN PATENT APPLICATION**

(43) Date of publication:
02.01.2004 Bulletin 2004/01

(51) Int Cl.7: **A61C 13/15**, B23K 26/06

(21) Application number: 03008607.8

(22) Date of filing: 15.04.2003

(84) Designated Contracting States:
AT BE BG CH CY CZ DE DK EE ES FI FR GB GR
HU IE IT LI LU MC NL PT RO SE SI SK TR
Designated Extension States:
AL LT LV MK

(30) Priority: 24.06.2002 US 178142

(71) Applicant: Coltene/Whaledent, Inc.
Mahwah, New Jersey 07430 (US)

(72) Inventor: Abdelqader, Steven
Thiells, New York 10984 (US)

(74) Representative: Grättinger & Partner (GbR)
Postfach 16 55
82306 Starnberg (DE)

(54) **LED curing light**

(57) A curing light includes a light source having an array of light emitting diodes (LEDs), the LEDs being held in a holder so that the emitters of the LEDs define a spherical surface having a known radius. Light is transmitted by the LED array to a receiver formed as a bundle of optical fibers. A receiving end of the receiver is positioned at distance from the array so that substantially all light emitted by the diodes is captured by the receiver. The bundle is drawn is such manner that it has a diameter of between 14 and 25 millimeters at a light receiving end, and a diameter of between 3 and 13 millimeters at a light transmitting end. A collimating lens is optionally interposed between the light source and the receiver.

81

80

82

*Fig. 4C*

## Description

## FIELD OF THE INVENTION

This invention relates to a light transmission system for curing instruments. More particularly, the invention relates to a light transmission system comprising an array of light emitting diodes (LEDs) optically coupled to a light guide arranged as a bundle of drawn optical fibers having a wide diameter at a light receiving end and a narrowed diameter at a light emitting end.

## BACKGROUND OF THE INVENTION

Dental composites employ well-known materials, and are used in a variety of dental procedures including restoration work and teeth filling after root canal procedures and other procedures requiring drilling. Several well-known dental composites have been sold, for example, under the trade names of BRILLIANT LINE, Z-100, TPH, CHARISMA and HERCULITE & BRODIGY.

These composites are typically formed from liquid and powder components that are mixed together to form a paste. The paste is formed to have a consistency sufficiently workable and self-supporting to be applied to an opening or cavity in a tooth. The liquid component may comprise phosphoric acid and water, while the powder component may comprise ceramic materials such as cordite, silica or silicium oxide. After the composite is applied to a tooth, it must be cured to form a permanent bond with the tooth.

Curing requires the liquid component to evaporate, causing the composite to harden. In the past, curing has been accomplished by air drying, which has had the disadvantage of requiring significant time. This time can greatly inconvenience the patient. More recently, light curing has become popular in the field of dentistry as a means for decreasing curing times. According to this trend, curing lights have been developed for dental curing applications. An example of such a curing light is illustrated by U.S. Patent No. 5,975,895, issued November 2, 1999 to Sullivan, which is hereby incorporated by reference.

Conventional dental curing lights generally employ tungsten filament halogen lamps that incorporate a filament for generating light, a reflector for directing light and a blue filter to limit transmitted light to wavelengths in the region of 400 to 500 nanometers (nm). Light is typically directed from the filtered lamp to a light guide, which directs the light emanating from a light emitting end of the guide to a position adjacent to the material to be cured.

Composites may be selected to take advantage of curing light properties. For example, for certain polymer composite filling materials, blue light provided by a curing light may be used to excite a camphoroquinine photo intitiator, which has a light absorption peak of 468 nm. This in turn stimulates the production of free radicals in a tertiary amine component, causing polymerization and hardening of the polymer composite.

A problem with conventional halogen-based lights is that the lamp, filter and reflector degrade over time. This degradation is particularly accelerated, for example, by the significant heat generated by the halogen lamp. For example, this heat may cause filters to blister and cause reflectors to discolor, leading to reductions in light output and curing effectiveness. While heat may be dissipated by adding a cooling fan to the light, this fan may cause other undesired effects (for example, undesirably dispersing a bacterial aerosol that may have been topically applied by the dentist to the patient's mouth). Alternate lamp technologies using Xenon and laser light sources have been investigated, but these technologies tend to be costly, require filtration, consume large amounts of power and generate significant heat. Laser technologies also require stringent safety precautions.

Alternatively, Light Emitting Diodes (LEDs) and Laser Diodes (LDs) appear to be good candidate curing light sources, having excellent cost and life characteristics. In addition, LEDs and LDs can be designed to produce a significant portion of light output having a frequency in the desired range of 400 to 500 nm, thereby eliminating the need to incorporate supplementary spectral filters in the device. For example, much of the spectral radiant intensity for many blue LEDs peaks at 468 nm, producing an almost ideal bandwidth of the required blue light. As a result, LED light sources require no filters and generate little waste heat, and are thereby capable of transferring a greater percentage of applied power to generating blue light than, for example, halogen light sources. Generating little heat, they also present less risk of irritation or discomfort to the patient.

To date, it has been difficult to generate sufficient power levels from LED or LD lamp designs for dental curing applications. A minimum of 800 milliwatts per square centimeter is required. Accordingly, it would be desirable to develop a curing light using LED or LD lamps having sufficient power to support dental curing applications.

## SUMMARY OF THE INVENTION

These and other deficiencies in the prior art have been remedied by a novel light source comprising an array of LEDs fixedly held in a LED holder such that emitters in each of the LEDs are approximately positioned along a spherical surface defined by a predetermined radius. The radius is selected in order to provided a desired focal length for the LED array.

In a first preferred embodiment of the present invention, the array comprises 36 LEDs and has a focal length of 0.445 inches.

In a second embodiment of the present invention, the LED array is combined with a light guide having a light receiving end positioned near the focal length of the LED array. The light guide comprises a bundle of optical fibers, which have been progressively drawn so that the diameter of the bundle at a receiving end is between 14 and 25 millimeters (mm), and the diameter of the bundle at a light emitting end is between 3 and 13 mm. The large diameter at the receiving end allows the receiving end to capture substantially all of the light emitted by the LED array while being positioned at a minimum distance from the LED array. Minimizing the distance between the LED array and light guide reduces the amount of light energy lost by attenuation over this distance.

In the second embodiment of the present invention, the surface of the receiving end of the light guide may be concave and, preferably, follow a spherical surface. This surface shape reduces reflections of light transmitted by the LED array, thereby capturing more of the transmitted light and reducing light energy losses.

In a third preferred embodiment of the present invention, a convex lens is interposed between the array and light guide of the second embodiment to further focus and curing light emitted by the LED array for transmission through the light receiving end of the light guide.

The aforementioned objects, features and advantages will, in part, be pointed out with particularity, and will, in part, become obvious from the following more detailed description of the invention, taken in conjunction with the accompanying drawing, which forms an integral part thereof. While the description describes the array as comprising a plurality of LEDs, the invention contemplates that a variety of other solid-state light sources may also be employed for this purpose (for example, laser diodes). Additionally, while the description describes applications of the light source relating to the curing of dental composites, the present invention contemplates a variety of other uses (for example, as a focused light source for microscopy applications).

## BRIEF DESCRIPTION OF THE DRAWINGS

A more complete understanding of the invention may be obtained by reading the following description of specific illustrative embodiments of the invention in conjunction with the appended drawing in which:

Figure 1 illustrates some general properties associated with light from a light source directed to an optical fiber;
Figure 2 illustrates a first example of a prior art curing light;
Figure 3 illustrates a second example of a prior art curing light;
Figures 4A - 4C show an embodiment of the LED array of the present invention;
Figures 5A, 5B illustrate an embodiment of the fiber bundle light guide employed by the present invention;
Figures 6A, 6B illustrate positioning of the light guides of Figures 5A, 5B with respect to the LED array of Figures 4A - 4C;
Figure 7 provides a cross-sectional view of a third embodiment of the preset invention employing a collimating lens for reducing focal distance between the LED array and the fiber bundle ; and
Figures 8A - 8C illustrate a preferred example of the third embodiment of Figure 7.

## DETAILED DESCRIPTION OF THE PREFERRED EMBODIMENTS

The following detailed description includes a description of the best mode or modes of the invention presently contemplated. Such description is not intended to be understood in a limiting sense, but to be an example of the invention presented solely for illustration thereof, and by reference to which in connection with the following description and the accompanying drawings one skilled in the art may be advised of the advantages and construction of the invention.

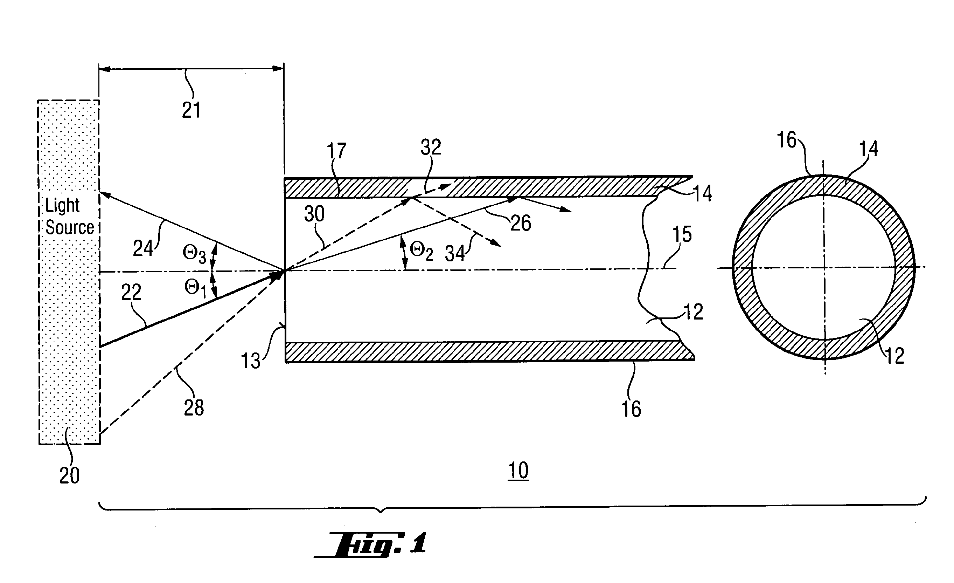

Figure 1 illustrates a standard light transport medium in the form of an optical fiber 10. Optical fiber 10 includes a fiber core 12, a fiber cladding 14 and a fiber outer coating 16. Fiber core 12 typically serves as the portion of the fiber operative to carry light, and has an index of refraction $N_1$. Fiber cladding 14 serves to help confine light within the core 12, and has an index of refraction $N_2$, which is typically less than $N_1$. Fiber Outer coating 16 provides protection against abrasion and other potential physical damage to fiber 10. A typical fiber 10 in the present inventive application might have an outer diameter between 0.001 and 0.003 inches in diameter, and have about 83 percent of its cross-sectional area comprising the core 12 and about 17 percent of its cross-sectional area comprising the cladding 14.

Incident beam 22 from light source 20 moves across air gap 21 to strike receiving end 13 of the fiber 10 at an angle $\theta_1$ with respect to fiber centerline 15. Incident beam 22 is reflected at face 13 as reflected beam 24, and is refracted at face 13 as refracted beam 30. Reflected beam 24 makes an angle $\theta_3$ with respect to centerline 15, and refracted beam 30 makes an angle $\theta_2$ with centerline 15. Because face 13 is perpendicular to centerline 15, angle $\theta_3$ is equal to angle $\theta_1$. Employing Snell's law, angle $\theta_2$ can be determined by using the following relationship:

$$N_{air} * \sin \theta_1 = N_{core} * \sin\theta_2 \tag{1}$$

Where $N_{air}$ is an index of refraction for air, and $N_{core}$ is an index of refraction for the fiber core.

As illustrated in Figure 1 by light beam 28, if $\theta_1$ becomes too large, a portion of refracted light beam 30 will be further refracted at interface 17 between core 12 and cladding 14 to exit the core as light beam 32. The angle beyond which light will not be fully carried in core 12 is referred to as the critical angle, and can be calculated from the associated indices of refraction. The sine of the critical angle is called numerical aperture, and may be calculated as follows:

$$\text{Numerical Aperture (NA)} = \sqrt{((N_{core})^2 - (N_{clad})^2)} \tag{2}$$

Where $N_{core}$ is the index of refraction for core 12, and $N_{clad}$ is the index of refraction for the cladding 14.

For example, in a common fiber configuration where $N_{core} = 1.62$ and $N_{clad} = 1.52$, NA = .56, which correspond to critical angle of 34 degrees. As the fiber 10 accordingly accepts light up to 34 degrees off centerline 15 in any direction, the acceptance angle of the fiber 10 is twice the critical angle, or 68 degrees. As optical fibers tend to preserve angle of incidence during propagation of light, light entering a fiber 10 will tend to exit the fiber at an angle equivalent to the angle of entry. Accordingly, the cone of light produced at the exit of the fiber will be limited to the smaller of the acceptance angle of the fiber 10 and an incident angle associated with light source 20.

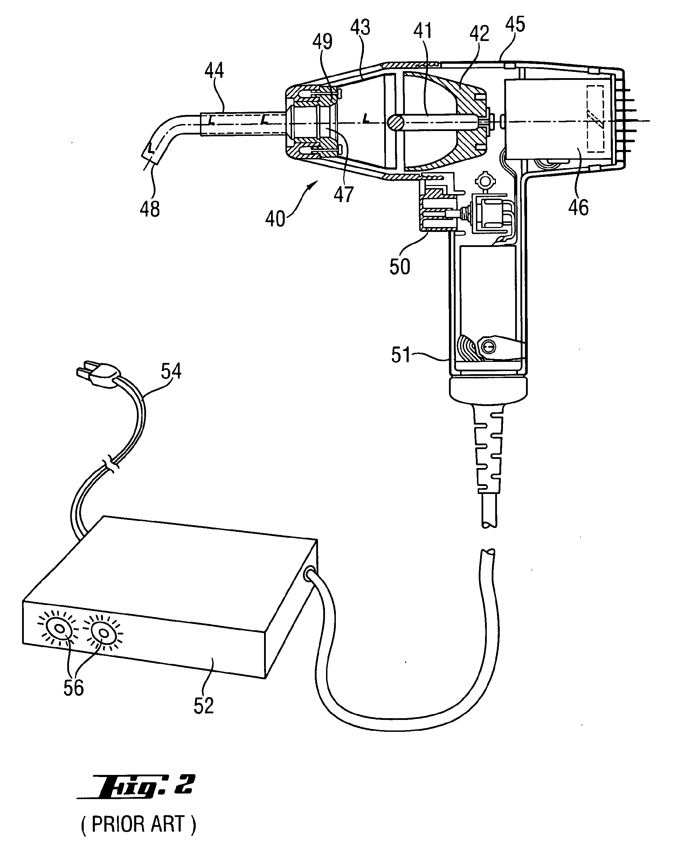

Figure 2 illustrates a conventional curing light 40 as disclosed by U.S. Patent No. 5,975,895. Curing light 40 includes a halogen lamp 41 that is mounted in a reflector 42. Light reflected by reflector 42 is contained by a collector 43, and directed to light receiving end 47 of fiber optic bundle 44 and then to light emitting end 48 of bundle 44. The light reflected from lamp 41 passes through a corrective filter 49 before entering receiving end 47. Lamp 41, reflector 42, collector 43, filter 49 and receiving end 47 of fiber optic bundle 44 are each aligned along a centerline of barrel 45. Lamp 41 is powered by power and control unit 52, cooled by a fan 46, and actuated by a switch 50 in handle 51. Power and control unit 52 includes AC power cord 54 and controls 56. Controls 56 may be used, for example, to control power output and timing of the curing light 40.

The curing light 40 of Figure 2 suffers from a number of the previously discussed difficulties associated with conventional curing lights. Halogen lamp 41 requires use of a costly, externally provisioned power supply 52 to power the light 40. Filter 49, lamp 41 and reflector elements 42, 43 are each subject to degradations over time. Lamp 41 produces a substantial amount of heat, necessitating both the addition of cooling fan 46 and the positioning of lamp 41 at a substantial distance from receiving end 47 of light guide 44 for patient safety.

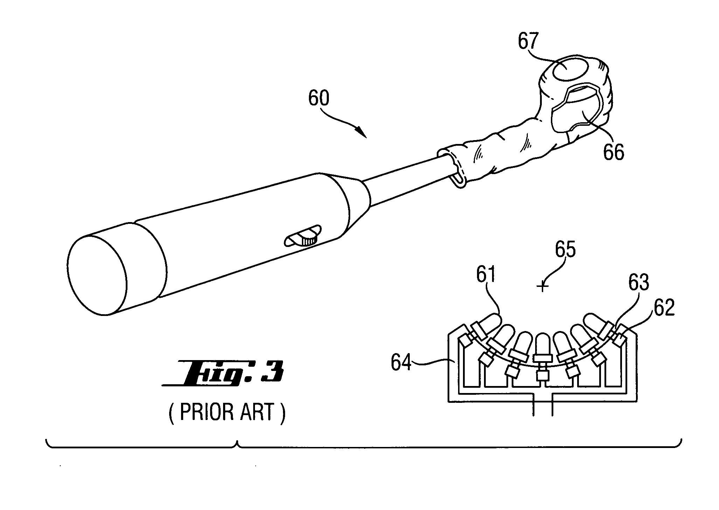

U.S. Patent No. 6,102,696 to Osterwalder et al. discloses an alternative curing light design using LEDs or LDs. As illustrated in Figure 3, the curing light 60 of Osterwalder includes, for example, a LED light source including a plurality of LEDs 61 arranged along a concave edge 63 of a circuit board 64, each LED being interconnected to the circuit board 64 at a connecting resistor 62. In this configuration, the light source produces a focused light beam having a focal point 65.

While the light source of curing light 60 solves some of the difficulties associated with other conventional curing lights, it exhibits certain other deficiencies. As the small number of LEDs employed in the light source generate a modest power level, the light source is positioned in an application end 66 of the curing light 60 so that the light can be transmitted over a short distance through a window 67. Because no light guide is employed in curing light 60, the application end 66 housing the light source must be placed in close proximity to the materials being cured. Application end 66 may be relatively large, and therefore difficult to use in applications having limited physical access such as teeth fillings.

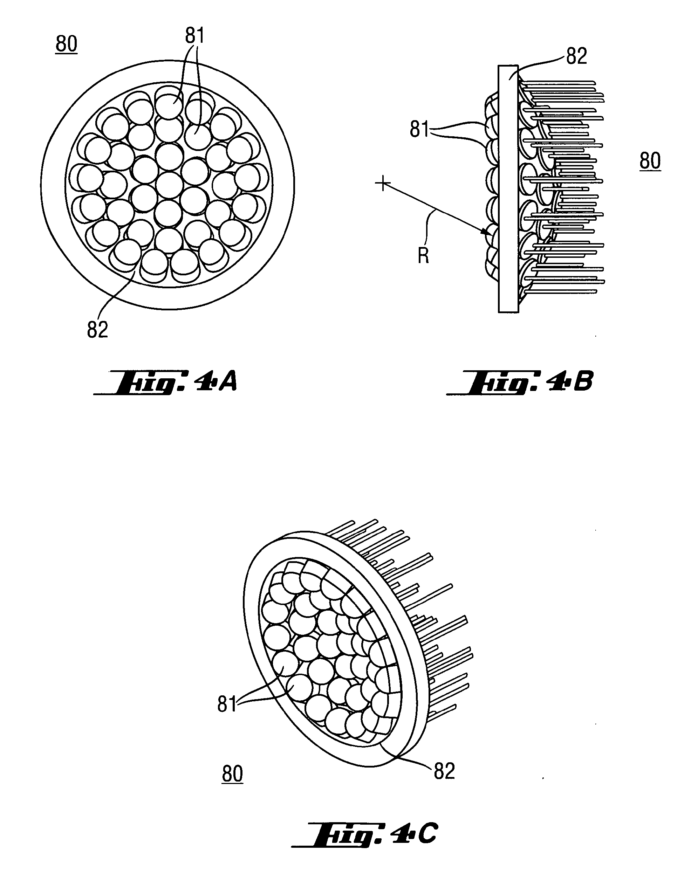

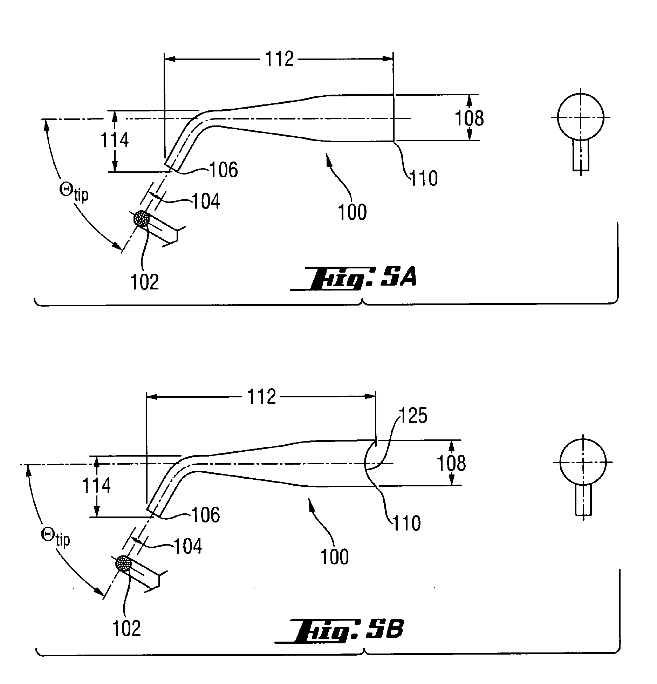

The limitations of the prior art are largely overcome by a novel LED light source 80 illustrated in Figures 4A - 4C, comprising a plurality of LEDs 81 and a LED holder 82 arranged for fixedly holding the plurality of LEDs 81 so that an emitter in each LED is approximately positioned on a spherical surface having a predetermined radius R. Figure 4A - 4C respectively illustrate top, side and perspective views of the novel LED light source 80. The radius R may be defined by the following relationship:

$$R = N * L^2 * f * x \tag{3}$$

Where N is equal to the number of diodes, L is a focusing distance of the light source, f is an average distance of each LED emitter from a face of its associated LED diode, and x is a correcting factor. L is preferably maintained between 0.400 inches and 0.600 inches. Applicant has successfully constructed LED light sources of this type that have included between 9 and 99 LEDs 81 in the LED holder 82. In a preferred embodiment of the present invention:

N=36,
F=0.198 inches
L=0.445 inches, and
X = 1

In the preferred embodiment, R can be calculated as 1.199.

LEDs 81 in light source 80 will naturally exhibit a variety of spectral characteristics as a result of variation in associated manufacturing processes. While each of the LEDs 81 are selected to transmit light that is primarily in a spectral range of 430 nanometers (nm) to 490 nm in wavelength, individual ones of LEDs 81 will vary as to characteristic wavelength (wavelength produced with greatest intensity) and spectral range. Accordingly, one aspect of the present invention provides for selectively positioning LEDs 81 within holder 82 in accordance with their spectral characteristics. In one embodiment of the present invention, individual LEDs 81 are grouped according to their spectral characteristics and are randomly selected from these groups and positioned in holder 82. This scheme provides for a reasonably uniform spectral range and intensity across the full area of the incident light beam generated by light source 80.

Alternatively, LEDs 81 may be grouped and selected so that LEDs having most desired spectral characteristics (for example, characteristic wavelength of 468 nm) occupy central positions on holder 82, and LEDs having least desired spectral characteristics occupy peripheral or outer positions on holder 82. Because peripherally-located LEDs may be positioned at or near the critical angle, this embodiment provides, for example, an incident light beam that maximizes transmission at the desired characteristic wavelength.

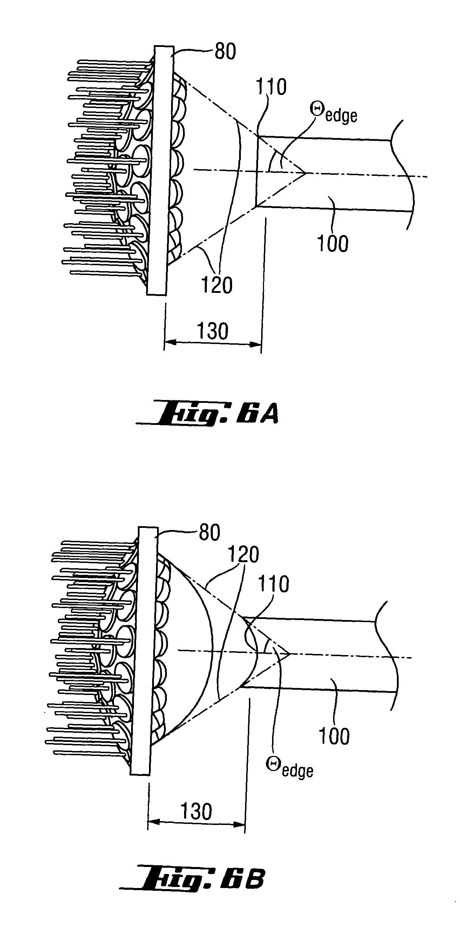

Another important element of the present invention comprises a novel light guide for directing light from the LED light source to an application. Figures 5A, 5B illustrate two examples of the novel light guide. Light guides 100 comprise a plurality of optical fibers 102 arranged in a bundle. Optical fibers 102 are heated and drawn so that a bundle diameter 104 at light emitting end 106 is substantially smaller than a bundle diameter 108 at light receiving end 110. Diameters for individual fibers in the bundle may typically range from 0.001 to 0.003 inches in diameter. As a result, light emitting end bundle diameter 104 preferably ranges between 3 and 13 millimeters, while receiving end bundle diameter 108 preferably ranges from 14 to 25 millimeters in diameter. Light receiving end bundle diameter 108 is accordingly substantially larger than bundle diameters found in conventional curing lamp guides.

Emitting end 106 of light guide 100 may be positioned at an angle with respect to receiving end 110 (defined between longitudinal axes of emitting end 106 and receiving end 110 by tip angle $\theta_{tip}$). Light guide 100 may, for example, have a typical length 112 of between two and eight inches and a typical tip depth 114 of between ½ and 3 inches.

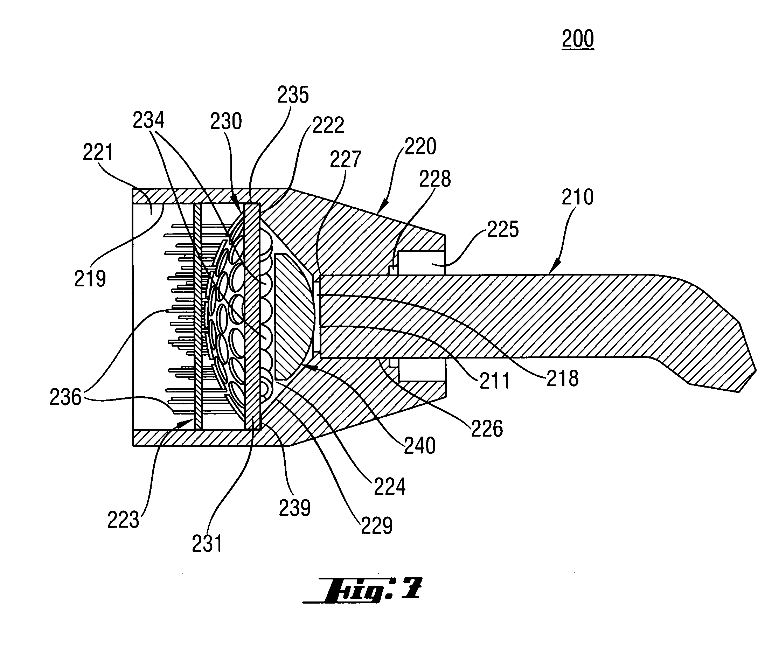

Receiving end bundle diameter 108 has the advantage of enabling light guide 100 to be closely positioned with respect to light source 80 (see, for example, Figures 6A, 6B). In Figure 6A, rays 120 represent an outer edge limit for incident light rays generated by the light source 80. Given that an associated outer edge angle $\theta_{edge}$ does not exceed a critical angle for the light guide 100, a minimum distance 130 between the light source 80 and receiving end 110 of light guide 100 is inversely related to the receiving end diameter 108 of the light guide 100. Thus, light guide 100 having an expanded receiving end diameter 108 can be positioned more closely to light source 80 than conventional light guides. As a result, less light energy is attenuated by air gap 21 as shown in Figure 1, thereby increasing light transmission through receiving end 108 of light guide 100 of Figure 6A.

A second example of light guide 100 is illustrated in Figures 5B and 6B. In Figure 5B, receiving end 110 of light guide 100 is formed to have a concave surface 125 that may be, for example, approximately spherical in shape. The concave surface 125 effectively alters the angle of refraction $\theta_2$ shown in Figure 1 so that the critical angle $\theta_1$ may be enlarged, and the amount of light reflected at angle of reflection $\theta_3$ may thereby reduced. As a result, comparing the light guide of Figure 5B to the light guide of Figure 5A, less light energy from light source 80 is reflected by receiving end 110, thereby increasing light transmission through receiving end 110.

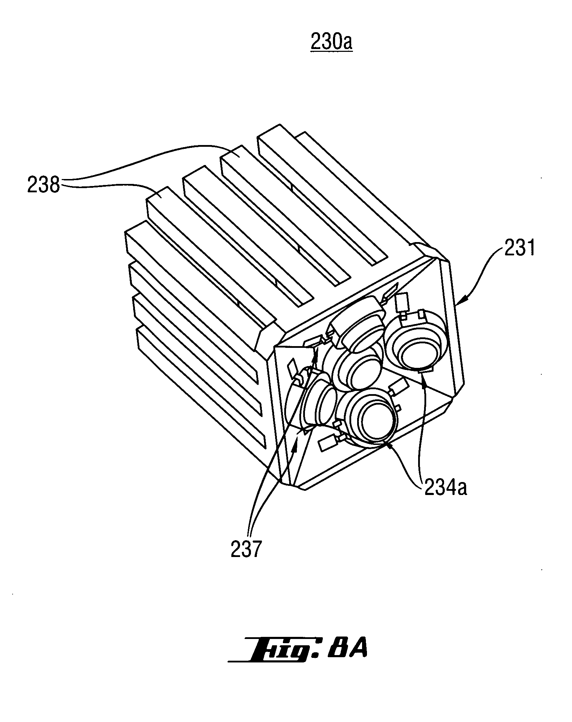

A third embodiment of the present invention is illustrated by LED curing light assembly 200 of Figure 7. Figure 7 presents a cross-sectional view of assembly 200, comprising light guide 210, light source 230, collimating lens 240 and assembly housing 220. Lens 240 is fixedly positioned between light source 230 and light guide 210, and acts to further collimate light emitted by light source 230 in order to reduce the focal distance between light source 230 and light guide 210. This reduction in focal distance helps to further reduce transmissive losses between light source 230 and light guide 210. Collimating lens 240 is preferably an anti-reflective fused silica convex lens having a minimum of 98% transmissivity within the operative spectral range (430 nm to 490 nm), as may be commercially obtained, for example, from Thermo Electron Corporation of Waltham, Massachusetts.

Light guide 210 is positioned through recess 225 and cavity 226 of housing 220 such that light receiving end 211 of light guide 210 is held against annular seat 227 of cavity 226. Recess 228 is arranged to hold an O-ring (not shown) for gripping an outer diameter of light guide 210. Recess 225 is arranged to engagingly receive a retaining nut (not shown) for applying sufficient lateral pressure to the O-ring in recess 228 to cause an inner diameter of the O-ring to meet and fixedly grip the outer diameter of light guide 210 in order to hold light guide 210 against annular seat 227 of cavity 226.

With reference to light source 230, a front annular surface 233 of holder 231 of light source 230 is fixedly held against seat 222 in cavity 221 of housing 220. A variety of conventional means may be employed to hold surface 233 against seat 222 including, for example, an interference fit between outer diameter 235 of holder 231 and inner surface 219 of cavity 221. Lens 240 may also be fixedly positioned by a variety of conventional means, including fixedly fitting lens 240 within cavity 224 in physical contact with conical surface 229 and covers of ones of the plurality of LEDs 234 in light source 230.

Mounting plate 223 is fixedly mounted within cavity 221 by one of a variety of conventional means. Mounting plate 223 includes a variety of apertures (not shown) for receiving terminals 236 of light source 230, and may further include printed wiring paths (not shown) for interconnecting certain ones of terminals 236.

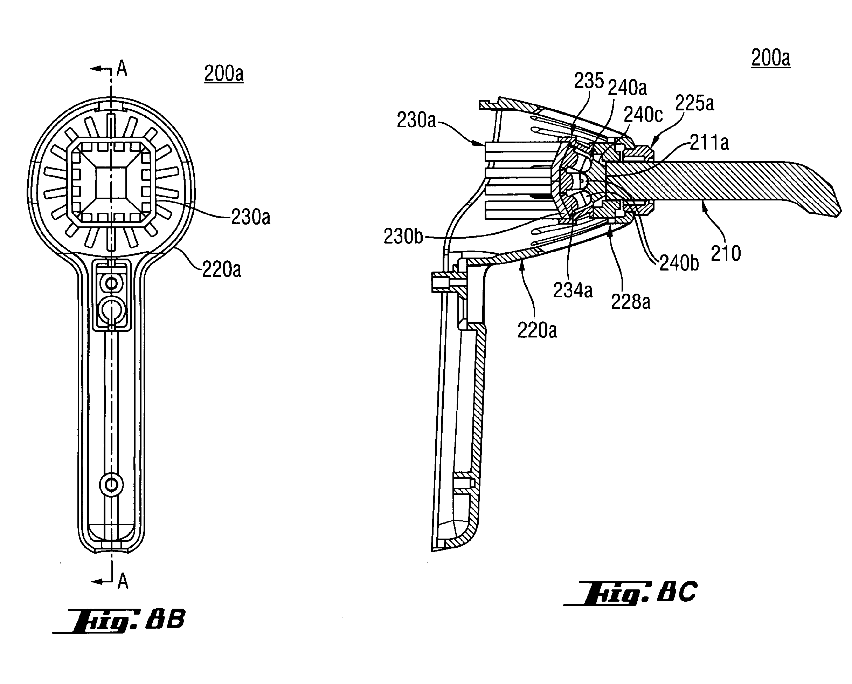

Figures 8A - 8C illustrate a preferred example of the third embodiment of Figure 7. Figure 8A provides a perspective view of a light source 230a comprising LEDs 234 each individually mounted on facets 237 of holder 231. Facets 237 are configured so that emitters associated with LEDs 234 are approximately positioned on a spherical surface. As shown in Figure 8A, light source 230a comprises five LEDs 234. Four of the five LEDs 234 at a periphery of holder 231 are positioned at an angle of approximately 25 degrees with respect to a fifth, centrally-located LED in order to define the approximately spherical surface.

In order to generate sufficient light energy for dental curing applications (an excess of 800 milliwatts of output power), LEDs 234 are high output (high luminous flux) LEDs generating in excess of 160 milliwatts of output power (commercially available, for example, as LUXEON LEDs from Lumileds Lighting, LLC of San Jose, California). To assist with dissipation of heat generated by LEDs 234, holder 231 is formed from a heat-conductive material (for example, aluminum) and incorporates fingers 238 that effectively operate as a heat sink.

Figures 8B and 8C provide cutaway views illustrating assembly 200a comprising light source 230 fixedly positioned in housing 220a. Figure 8C presents a cross-sectional view of assembly 200a through section A-A of Figure 8B. As illustrated in Figure 8C, light source 230a is fixedly held at a desired position in housing 220a by front cup 235. Front cup 235 provides a friction fit against a perimeter 230b of light source 230a, and may comprise a variety of materials including natural rubber and plastic. Light guide 210 is fixedly held in housing 200a by bushing 228a, which applies force against an outer surface of light guide 210 when compressed by front cup clamp 225a.

Collimating lens 240a is interposed between light source 230a and light guide 210. Light receiving end 211 has a concave surface 211a for matingly receiving convex surface 240c of lens 240a. An opposing surface of lens 240a includes pockets 240b for matingly receiving dome portions of LEDs 234a. In this configuration, a viewing angle of approximately 110 degrees for LEDs 234a is collimated by lens 240 into a viewing angle of approximately 15 degrees for light rays leaving lens 240a and entering light guide 210

Those skilled in the art will recognize a variety of additional embodiments of the present invention are not described, but are contemplated within the scope of the invention. For example, one skilled in the art could readily envision constructing light source 80 with a plurality of LDs rather than a plurality of LEDs.

While the present invention has been described at some length and with some particularity with respect to the several described embodiments, it is not intended that it should be limited to any such particulars or embodiments or any particular embodiment, but it is to be construed with references to the appended claims so as to provide the broadest possible interpretation of such claims in view of the prior art and, therefore, to effectively encompass the intended scope of the invention.

**Claims**

1. A concentrated light source, the light source comprising:

   a plurality of light emitting elements (LEMs); and
   a holder for fixedly holding the plurality of LEMs, said holder holding the LEMs such that each of the plurality of LEMs is approximately positioned on a spherical surface having a predetermined radius.

2. The light source of claim 1, wherein the LEMs are selected from the group consisting of light emitting diodes (LEDs) and laser diodes (LDs).

3. The light source of claim 1, wherein the number of LEMs in the plurality of LEMs is greater than 8.

4. The light source of claim 3, wherein the number of LEMs in the plurality of LEMs is less than 99.

5. The light source of claim 2, wherein the light source comprises 36 LEDs.

6. The light source of claim 1, wherein the predetermined radius is determined as the product of a number representing the plurality of LEMs, the square of a selected focal length, an average distance between an emitter and external face of the plurality of LEMs, and a corrective factor.

7. The light source of claim 6, wherein the selected focal length is between 0.400 and 0.600 inches, the average distance is 0.198 inches and the corrective factor is 1.0.

8. The light source of claim 7, wherein the number of LEMs is 36 and the predetermined radius as measured from an emitter surface in each of the plurality of LEMs is 1.199 inches.

9. The light source of claim 1, wherein each of the plurality of LEMs emits light having wavelengths substantially limited to a predetermined spectral range.

10. The light source of claim 2, wherein each of the plurality of LEMs is a LED emitting light at wavelengths substantially between 430 and 490 nanometers.

11. The light source of claim 10, wherein each of the plurality of LEDs may be grouped into two or more groups according to its characteristic wavelength and spectral range.

12. The light source of claim 11, wherein LEDs from the two or more groups are randomly positioned in the holder.

13. The light source of claim 11, wherein LEDs from a selected one of the two or more groups are positioned in a central region of the holder.

14. The light source of claim 13, wherein LEDs from the selected one group exhibit a transmissive intensity peak at a wavelength of approximately 468 nanometers.

15. A curing light, the curing light comprising:

a light source having a plurality of light emitting elements (LEMs), wherein each of the plurality of LEMs is approximately positioned on a spherical surface having a predetermined radius; and
a light receiver, wherein the light receiver is positioned at a predetermined focusing distance from the light source, such that substantially all light energy emitted by the light source is captured by the light receiver at a receiving end.

16. The curing light of claim 15, wherein the LEMs are selected from the group consisting of light emitting diodes (LEDs) and laser diodes (LDs).

17. The curing light of claim 15, further comprising a collimating lens interposed between the light source and the light receiver.

18. The curing light of claim 17, wherein the lens is a convex lens comprising fused silica.

19. The curing light of claim 18, wherein the lens has a transmissivity of at least 98 percent for a spectral wavelength range between 430 nanometers (nm) and 490 nm.

20. The light source of claim 15, wherein the predetermined radius is determined as the product of a number representing the plurality of LEMs, the square of a selected focal length, an average distance between an emitter and external face of the plurality of LEMs, and a corrective factor.

21. The light source of claim 20, wherein the selected focal length is between 0.400 and 0.600 inches, the average distance is 0.198 inches and the corrective factor is 1.0.

22. The light source of claim 21, wherein the number of LEMs is 36 and the predetermined radius as measured from an emitter surface in each of the plurality of LEMs is 1.199 inches.

23. The curing light of claim 15, wherein the light receiver compnses a fiber optic bundle having a diameter of at least 14 millimeters at the receiving end.

24. The curing light of claim 23, wherein individual fibers in the bundle each have a numerical aperture of between 0.4 and 0.6.

25. The curing light of claim 24, wherein the numerical aperture is about 0.56.

26. The curing light of claim 23, wherein the bundle has a diameter no greater than 25 millimeters.

27. The curing light of claim 15, wherein the receiving end has a concave surface for receiving light energy.

28. The curing light of claim 27, wherein the concave surface is spherically shaped.

29. The curing light of claim 15, wherein the receiving end has a substantially flat surface for receiving light energy.

30. The curing light of claim 15, wherein the predetermined radius is determined as a function of a number of LEMs included in the light source and the square of the predetermined focusing distance.

31. The curing light of claim 23, wherein the fiber optic bundle is drawn toward an emitting end such that a diameter of the emitting end is no greater than 13 millimeters.

32. The curing light of claim 31, wherein the diameter of the emitting end is at least 3 millimeters.

33. The curing light of claim 15, wherein a longitudinal axis drawn through the emitting end forms an acute angle with a longitudinal axis drawn through the receiving end.

34. The curing light of claim 33, wherein the angle is no greater than 60 degrees.

35. A curing light, the curing light comprising:

a light source having a plurality of light emitting elements (LEMs), each LEM fixedly mounted in a holder such that each of the plurality of LEMs is approximately positioned on a spherical surface;
a collimating lens having a plurality of cup-shaped recesses in a light receiving surface, each of said plurality of cup-shaped recesses for matingly receiving a dome of one of the plurality of LEMs, said collimating lens further having a convex light transmitting surface opposite to said light receiving surface; and
a light guide having a concave light receiving surface for matingly receiving the convex light transmitting surface of said collimating lens;

wherein substantially all light energy emitted by the light source is captured by the light guide at the light receiving surface.

36. The curing light of claim 35, wherein the holder effectively operates as a heat sink for the plurality of LEMs.

37. The curing light of claim 36, wherein the holder comprises a heat-conducting material.

38. The curing light of claim 37, wherein the holder comprises aluminum.

39. The curing light of claim 36, wherein the retained further comprises a plurality of fingers extending rearward from a periphery of the holder.

40. The curing light of claim 35, wherein the plurality of LEMs comprise five light emitting diodes (LEDs), in combination generating at least 800 milliwatts of output power.

41. The curing light of claim 35, wherein the collimating lens comprises fused silica.

**42.** The curing light of claim 41, wherein the collimating lens has a transmissivity of at least 98% for a spectral wavelength range between 430 nanometers (nm) and 490 nm.

**43.** The curing lamp of claim 35, wherein the convex light transmitting surface is substantially spherical in shape.

**44.** The curing light of claim 35, wherein the light guide comprises a fiber optic bundle having a diameter of at least 14 millimeters proximate to the concave light receiving surface.

**Fig. 1**

**Fig. 2**

( PRIOR ART )

**Fig. 3**

( PRIOR ART )

**Fig. 4A**

**Fig. 4B**

**Fig. 4C**

**Fig. 5A**

**Fig. 5B**

*Fig. 6A*

*Fig. 6B*

230a

238

231

234a

237

Fig. 8A

Fig. 8C

Fig. 8B

# EP 1 374 797 A1

## EUROPEAN SEARCH REPORT

**European Patent Office**

**Application Number**

EP 03 00 8607

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | PATENT ABSTRACTS OF JAPAN<br>vol. 2000, no. 13,<br>5 February 2001 (2001-02-05)<br>& JP 2000 271155 A (USHIO INC),<br>3 October 2000 (2000-10-03) | 1,3,4,9,<br>10,15 | A61C13/15<br>B23K26/06 |
| Y | * abstract; figures 1-3 * | 16,17,<br>23,29,<br>31,32,<br>35-39,<br>43,44 | |
| Y | WO 00 67048 A (PREMIER LASER SYSTEMS INC)<br>9 November 2000 (2000-11-09)<br><br>* page 5, line 28 - page 11, line 20 *<br>* page 14, line 3 - line 17 *<br>* page 15, line 3 - line 10 *<br>* claims 1,3,7,15-19,29,47-51 *<br>* figures 2-5B * | 16,17,<br>23,29,<br>31,32,<br>35-39,<br>43,44 | |
| Y | WO 02 11640 A (KERR CORP)<br>14 February 2002 (2002-02-14)<br>* claims 3,24,37 * | 17 | **TECHNICAL FIELDS SEARCHED (Int.Cl.7)**<br><br>A61C<br>B23K |
| X,D | US 6 102 696 A (OSTENWALDER ET AL.)<br>15 August 2000 (2000-08-15)<br>* the whole document * | 1-4,9,10 | |
| X | PATENT ABSTRACTS OF JAPAN<br>vol. 1997, no. 05,<br>30 May 1997 (1997-05-30)<br>& JP 09 010238 A (OSADA RES INST LTD),<br>14 January 1997 (1997-01-14)<br>* abstract; figures * | 1,3,4,9,<br>10,15 | |

The present search report has been drawn up for all claims

| Place of search | Date of completion of the search | Examiner |
|---|---|---|
| THE HAGUE | 17 September 2003 | Raybould, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

19

# EP 1 374 797 A1

## ANNEX TO THE EUROPEAN SEARCH REPORT
## ON EUROPEAN PATENT APPLICATION NO.

EP 03 00 8607

This annex lists the patent family members relating to the patent documents cited in the above-mentioned European search report.
The members are as contained in the European Patent Office EDP file on
The European Patent Office is in no way liable for these particulars which are merely given for the purpose of information.

17-09-2003

| Patent document cited in search report | | Publication date | Patent family member(s) | | Publication date |
|---|---|---|---|---|---|
| JP 2000271155 | A | 03-10-2000 | NONE | | |
| WO 0067048 | A | 09-11-2000 | US | 6439888 B1 | 27-08-2002 |
| | | | AU | 4698100 A | 17-11-2000 |
| | | | CA | 2370456 A1 | 09-11-2000 |
| | | | EP | 1180983 A2 | 27-02-2002 |
| | | | WO | 0067048 A2 | 09-11-2000 |
| | | | US | 2002182563 A1 | 05-12-2002 |
| WO 0211640 | A | 14-02-2002 | AU | 8107401 A | 18-02-2002 |
| | | | BR | 0112973 A | 08-07-2003 |
| | | | EP | 1304977 A2 | 02-05-2003 |
| | | | WO | 0211640 A2 | 14-02-2002 |
| US 6102696 | A | 15-08-2000 | NONE | | |
| JP 09010238 5 | A | | NONE | | |

EPO FORM P0459

For more details about this annex : see Official Journal of the European Patent Office, No. 12/82

20